# EUROPEAN PATENT APPLICATION

(11) **EP 3 786 966 A1**
(43) Date of publication of application: **03.03.2021**
(21) Application number: 20192594.8
(22) Date of filing: 25.08.2020
(51) Int. Cl.: G16H 10/40

(54) **AUTOMATED VALIDATION OF MEDICAL DATA**

(30) Priority: 26.08.2019 WO PCT/CN2019/102636
(71) Applicant: F. Hoffmann-La Roche AG, 4070 Basel (CH); Qilu Hospital of Shandong University, Ji'nan 250012 (CN)
(72) Inventor: Liu, Daquan, P.R. Shanghai, 200335 (CN); Qian, Yin, P.R. Shanghai, 200335 (CN); Tao, Xiaojun, P.R. Shanghai, 200335 (CN); Wang, Hongchun, Ji'nan, 250012 (CN); Xing, Weibin, P.R. Shanghai, 200335 (CN); Zhang, Chenxi, P.R. Shanghai, 200335 (CN); Zhang, Yi, Ji'nan, 250012 (CN); Zhou, Qi, P.R. Shanghai, 200335 (CN)
(74) Representative: Altmann Stößel Dick Patentanwälte PartG mbB

(57) **Abstract**

Embodiments of the present disclosure relate to automated validation of medical data. Some embodiments of the present disclosure provide a method for medical data validation. The method comprises obtaining target medical data generated in a medical test and obtaining a machine learning model for validating medical data. The machine learning model represents an association between the medical data and validation results, the validation results indicating information about predetermined actions to be performed on the medical data. The method further comprises determining a target validation result for the target medical data by applying the target medical data to the machine learning model, the target validation result indicating information about a target action selected from the predetermined actions to be performed on the target medical data. Through the solution, it is possible to achieve automated medical data validation with high accuracy and efficiency as well as reduced manual efforts.

## Description

### FIELD

Embodiments of the present disclosure generally relate to the field of computer science and in particular, to methods, devices, and computer program products for automated validation of medical data.

### BACKGROUND

Medical tests are performed almost every day in medical laboratories and a large amount of medical test reports are generated therefrom to present medical data. Before releasing the medical test reports to clinical departments or patients, validation procedures are initiated to ensure that the medical data presented in the reports are valid so as to avoid erroneous diagnoses on patients. However, lots of labor efforts are required in current validation procedures even though some automated functions have been introduced.

Currently, a rule-based engine is used to validate a medical test report. The rule-based engine is configured to validate whether the medical test report can be directly passed to a clinical department or the patient by determining whether test results in the report satisfy predetermined rules. The medical test report that fails to satisfy one or more of the predetermined rules will be provided for laboratory experts to review manually with their medical experience and knowledge.

Even with support of the current rule-based function, a large quantity of manpower has to be paid for the validation of medical data. In addition to the human resource cost, validation accuracy and efficiency may vary depending on experience and professional level of laboratory technicians. Therefore, it is desired to obtain a solution for automated validation of medical data to achieve high accuracy and efficiency with reduced manual efforts.

### SUMMARY

In general, example embodiments of the present disclosure provide a solution for automated validation of medical data.

In a first aspect, there is provided a method for medical data validation. The method comprises obtaining target medical data generated in a medical test and obtaining a machine learning model for validating medical data. The machine learning model represents an association between the medical data and validation results, and the validation results indicate information about predetermined actions to be performed on the medical data. The method further comprises determining a target validation result for the target medical data by applying the target medical data to the machine learning model, the target validation result indicating information about a target action selected from the predetermined actions to be performed on the target medical data.

In a second aspect, there is provided a method of providing a machine learning model for validating medical data. The method comprises obtaining training data comprising historical medical data and associated labeling information. The labeling information indicates predetermined actions performed on the historical medical data. The method further comprises generating a first machine learning model for validating medical data based on the training data such that the first machine learning model represents an association between the medical data and validation results indicating information about the predetermined actions to be performed on the medical data.

In a third aspect, there is provided an electronic device. The electronic device comprises at least one processor; and at least one memory comprising computer readable instructions which, when executed by the at least one processor of the electronic device, cause the electronic device to perform the steps of the method in the first aspect described above.

In a fourth aspect, there is provided an electronic device. The electronic device comprises at least one processor; and at least one memory comprising computer readable instructions which, when executed by the at least one processor of the electronic device, cause the electronic device to perform the steps of the method in the second aspect described above.

In a fifth aspect, there is provided a computer program product. The computer program product comprises instructions which, when executed by a processor of an apparatus, cause the apparatus to perform the steps of any one of the methods in the first aspect described above.

In a sixth aspect, there is provided a computer program product. The computer program product comprises instructions which, when executed by a processor of an apparatus, cause the apparatus to perform the steps of any one of the methods in the second aspect described above.

It is to be understood that the summary section is not intended to identify key or essential features of embodiments of the present disclosure, nor is it intended to be used to limit the scope of the present disclosure. Other features of the present disclosure will become easily comprehensible through the following description.

### BRIEF DESCRIPTION OF THE DRAWINGS

The following detailed description of the embodiments of the present disclosure can be best understood when read in conjunction with the following drawings, where:
Fig. 1 illustrates an example environment in which embodiments of the present disclosure may be implemented;
Fig. 2 illustrates a block diagram of a system for medical data validation according to some embodiments of the present disclosure;
Fig. 3 illustrates a block diagram of the computing device for training of one or more machine learning models in the system of Fig. 2 according to some embodiments of the present disclosure;
Fig. 4 illustrates an example clustering result for a set of historical medical data according to some embodiments of the present disclosure;
Fig. 5 illustrates a block diagram of the computing device for application of one or more machine learning models in the system of Fig. 2 according to some embodiments of the present disclosure;
Fig. 6 illustrates an example medical test report according to some embodiments of the present disclosure;
Fig. 7 illustrates a block diagram of the computing device for training of one or more machine learning models in the system of Fig. 2 according to some other example embodiments of the present disclosure;
Fig. 8 illustrates a block diagram of a system where one or more machine learning models are compatible with a rule-based engine and a laboratory information system (LIS) according to some embodiments of the present disclosure;
Fig. 9 illustrates a flowchart of an example process for validating medical data according to some embodiments of the present disclosure;
Fig. 10 illustrates a flowchart of an example process for providing a machine learning model for validating medical data according to some embodiments of the present disclosure; and
Fig. 11 illustrates a block diagram of an example computing system/device suitable for implementing example embodiments of the present disclosure.

Throughout the drawings, the same or similar reference numerals represent the same or similar element.

### DETAILED DESCRIPTION

Principle of the present disclosure will now be described with reference to some embodiments. It is to be understood that these embodiments are described only for the purpose of illustration and help those skilled in the art to understand and implement the present disclosure, without suggesting any limitation as to the scope of the disclosure. The disclosure described herein can be implemented in various manners other than the ones described below.

In the following description and claims, unless defined otherwise, all technical and scientific terms used herein have the same meaning as commonly understood by one of ordinary skills in the art to which this disclosure belongs.

References in the present disclosure to "one embodiment," "an embodiment," "an example embodiment," and the like indicate that the embodiment described may include a particular feature, structure, or characteristic, but it is not necessary that every embodiment includes the particular feature, structure, or characteristic. Moreover, such phrases are not necessarily referring to the same embodiment. Further, when a particular feature, structure, or characteristic is described in connection with an example embodiment, it is submitted that it is within the knowledge of one skilled in the art to affect such feature, structure, or characteristic in connection with other embodiments whether or not explicitly described.

It shall be understood that although the terms "first" and "second" etc. may be used herein to describe various elements, these elements should not be limited by these terms. These terms are only used to distinguish one element from another. For example, a first element could be termed a second element, and similarly, a second element could be termed a first element, without departing from the scope of example embodiments. As used herein, the term "and/or" includes any and all combinations of one or more of the listed terms.

The terminology used herein is for the purpose of describing particular embodiments only and is not intended to be limiting of example embodiments. As used herein, the singular forms "a", "an" and "the" are intended to include the plural forms as well, unless the context clearly indicates otherwise. It will be further understood that the terms "comprises", "comprising", "has", "having", "includes" and/or "including", when used herein, specify the presence of stated features, elements, and/or components etc., but do not preclude the presence or addition of one or more other features, elements, components and/ or combinations thereof.

### Example Environment

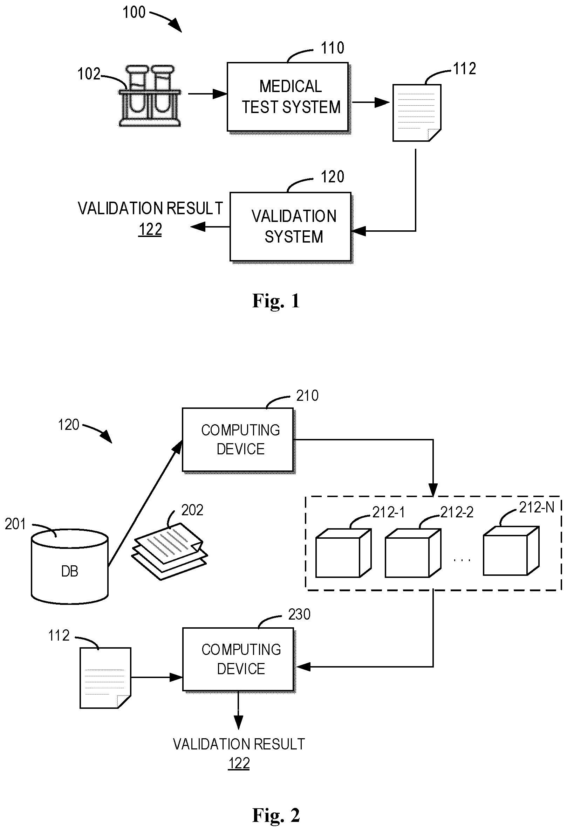

As mentioned above, validation procedures are important to ensure validity of medical data that are generated in various medical tests. Fig. 1 illustrates an environment 100 in which example embodiments of the present disclosure can be implemented. The environment 100 involves a typical workflow for medical diagnostic testing. The workflow generally includes performing a medical test on a test sample for medical diagnostics, generating medical data in the medical test, and validating the generated medical data.

In the environment 100 shown in Fig. 1, a medical test system 110 is configured to perform a medical test on a test sample 102 and generate medical data 112 associated with the test sample 102. The medical test may include an in-vitro diagnostic test, such as a biochemical detection test or an immuno-detection test. The medical test system 110 may include one or more automated laboratory instruments or analytical apparatuses designed for analysis of test samples via various chemical, biological, physical, or other medical test procedures. In some examples, the instruments or analytical apparatuses can be configured to induce a reaction of a sample with a reagent for obtaining a measurement value. Examples of such instruments or analytical apparatuses are clinical chemistry analyzers, coagulation analyzers, immunochemistry analyzers, hematology analyzers, urine analyzers and nucleic acid analyzers that are used for the qualitative and/or quantitative detection of analytes present in the samples, to detect the result of chemical or biological reactions and/or to monitor the progress of chemical or biological reactions.

The medical test system 110 may be operable to perform a medical test to measure the parameters of the sample or at least one analyte thereof. The medical test may involve one or more test items conducted on the sample 102. The medical test system 110 may return test results corresponding to respective test items as the medical data 112. Possible test results returned by the medical test system 110 may be obtained by determining concentrations of the analyte in the sample, a digital (yes or no) result indicating existence of the analyte in the sample (corresponding to a concentration above the detection level), data obtained from mass spectroscopy of proteins or metabolites and physical, mechanical, optical, electrical or chemical parameters of various types, and/or the like.

Some specific examples of types of test items may include levels of alanine aminotransferase (ALT), aspartate aminotransferase (AST), glutamic dehydrogenase (GLDH), concentration of sodium (NA), age, hemoglobin, plasma protein, albumin(ALB), globulin (GLB), total bilirubin (TBIL), direct bilirubin (DBIL), total bile acid (TBA), blood urea nitrogen (BUN), and so on. The examples listed here are not exhaustive. The test items to be performed in a specific medical test may be specified by an entity who requests the medical test, such as a clinic department, a physical examination center, a doctor, a patient, or the like.

The test sample 102 may also be referred to as a biological sample, which is a biological material(s) suspected of containing one or more analytes of interest and whose detection, qualitative and/or quantitative may be associated to a clinical condition. The biological sample is derived from a biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cells, or the like. Such biological source may be collected from a biological object, for example, a patient, a person, an animal, or the like.

The biological sample can be pretreated prior to use, such as preparing plasma or serum from blood. Methods of treatment can involve centrifugation, filtration, distillation, dilution, concentration and/or separation of sample components including analytes of interest, inactivation of interfering components, and addition of reagents. A biological sample may be used directly as derived from the source or used following a pretreatment to modify the character of the sample. In some embodiments, an initially solid or semi-solid biological material can be rendered liquid by dissolving or suspending it with a suitable liquid medium.

The term "reagent" refers to a substance which is added to a biological sample when performing a particular medical test on the biological sample to elicit a particular reaction in the sample. The reagents can be specific for a particular test or assay. For example, in a situation where a partial thromboplastic time of a blood sample shall be determined, the analyzer can be configured to add an activator as reagent to the blood sample to activate the intrinsic pathway of coagulation. Particular substances can be "modifying agents" or "reagents" in different situations. In some examples, a reagent may not be added to the biological sample to be tested.

The medical data 112 associated with the test sample 102 may include one or more test results of test items conducted in the medical test at the medical test system 110. The types of test results may be specified by an operator of the medical test system 110 (for example, a laboratory technician) or otherwise automatically identified from an electronic order via an information system connected with the medical test system 110. In some examples, the medical data 112 may be organized in a medical test report with specific test items and corresponding test results listed thereon. In some examples, in addition to the test results generated in the medical test, the medical data 112 may also include auxiliary information, such as information related to the test sample 102 and/or the biological object (such as the patient) from which the test sample 102 is collected.

The medical data 112 is provided to a validation system 120 to evaluate validity of the medical data 112 and determine whether the medical data 112 can be released or not. The need for validation is present because many problems can occur during the sample gathering and testing process. For example, a patient sample may be mislabeled, resulting in test results being reported in association with the wrong patient. As another example, the patient sample may have been improperly drawn or improperly handled, resulting in sample contamination and erroneous test results. Furthermore, a laboratory analyzer may be either malfunctioning or drifting out of calibration, again causing the analyzer to report erroneous results.

The validation system 120 provides a validation result 122 for the medical data 112. If the medical data 112 passes the validation, the validation result 122 may indicate that the medical data 112 is valid enough to be directly released (or released after a simple manual review) to a requestor who orders the medical test. If the medical data 112 is determined to be suspicious or abnormal, the validation result 122 may indicate that the medical data 112 fails the validation. In this case, further actions may need to be taken on the medical data 112. The validation result 122 as well as the medical data 112 may be provided to an information system, such as a laboratory information system (LIS), for archiving, recording, presentation, further processing, and/or the like. Following manual efforts may or may not be involved depending on the validation result 122.

In current validation procedures, a rule-based engine has been proposed to validate a medical test report. The rule-based engine is configured to validate whether the medical test report can be directly passed to the clinical department or the patient by determining whether test results in the report satisfy predetermined rules. Such rules are generally set as normal value ranges for respective test items. If one or more of the predetermined rules are not satisfied, the medical test report will be provided for laboratory experts to review manually with their medical experience and knowledge. The whole procedure is error prone and incurs high human resource/time consumption. Therefore, it is desired to obtain a solution for automated validation of medical data to achieve high accuracy and efficiency with reduced manual efforts.

### Working Principle and Example Validation System

According to example embodiments of the present disclosure, there is proposed a solution for automated validation of medical data. In this solution, one or more machine learning models are learned from historical medical data and associated labeling information indicating predetermined actions performed on the historical medical data. A machine learning model represents an association between the medical data and validation results. The one or more machine learning models can be utilized to validate target medical data and output a target validation result which indicates information about one of the predetermined actions to be performed on the target medical data. Through the solution, information can be learned from historical data to implement highly automated validation of medical data, which can thus significantly reduce manual efforts paid in reviewing the medical data and improve accuracy and quality in medical data validation.

In the following, example embodiments of the present disclosure are described with reference to the drawings. Reference is first made to Fig. 2, which illustrates a system for medical data validation according to some embodiments of the present disclosure. The system in Fig. 2 can be implemented as the validation system 120 in Fig. 1 and is used in the validation phrase of the whole workflow for medical diagnostic testing. The validation system 120 may also be referred to as an auto-validation system 120.

According to embodiments of the present disclosure, one or more machine learning techniques are employed to automatically analyze and validate medical data with human-like accuracy. The machine learning techniques may also be referred to as artificial intelligence (AI) techniques. In general, a machine learning model can be built, which receives input information and makes predictions based on the input information. For example, a classification model may predict a class of the input information among a predetermined set of classes.

Performing machine learning usually involves the following three phrases: a training phase to train a machine learning model with a training dataset by pairing an input with an expected output; an evaluation/test phase to estimate how well the model has been trained by estimating model performance characteristics (e.g., classification errors for classifiers, etc.) using an evaluation dataset and/or a test dataset; and an application phrase to apply the real-world data to the trained machine learning model to get the results. In the validation system 120 shown in Fig. 2, a computing device 210 is configured to implement the training phase and possibly the evaluation/test phase of machine learning, while a computing device 230 is configured to implement the application phrase of machine learning. It would be appreciated that although being illustrated separately, the computing devices 210 and 230 can be implemented as a single physical device to perform their functionalities described herein.

Specifically, the computing device 210 is configured to train one or more machine learning models, for example, machine learning models 212-1, 212-2, ..., 212-*N*, for validating medical data. *N* is any integer that is equal to or larger than one. The machine learning models 212-1, 212-2, ..., 212-*N* are collectively or individually referred to as machine learning models 212 hereinafter. The machine learning model(s) 212 are trained to automatically process input medical data and output a validation result for the input medical data. The trained machine learning model(s) 212 each represents an association between the medical data and the validation result. The association makes it possible to lead a machine learning model 212 to predict a suitable validation result for input medical data.

The medical data under validation may be generated in a medical test of a test sample and may be organized, for example, in a form of medical test report. The medical data includes one or more test results of test items, which may include measure values related to the test items and/or a digital (yes or no) result indicating existence of a certain analyte in the test sample. A validation result indicates information about an action to be performed on the medical data, which can be considered as a suggestion for the system or a user to automatically or manually decide how the medical data can be treated in a next step of the whole medical diagnostic testing workflow. The action is selected by the machine learning model 212 from a plurality of predetermined actions that are suitable for following processing of the medical data.

The validation procedure is to find potential errors in the medical data before the medical data is released to an entity who requests the medical test (such as the clinical department or the patient). If the medical data is validated as correct and having no error, the next step for the medical data is to release the medical data to that entity (or to require a quick manual review and then release to the entity). In this case, one possible action to be performed on the medical data is to release the medical data to an entity who requests the medical test related to the medical data directly or after a quick manual review. For convenience of discussion, this action is sometimes referred to as a first action herein.

In other cases, the medical data is validated as having an error, which may be due to the test sample, the performed medical diagnostic testing procedures, the reagent used in the medical test, mismatching with the physical condition of the biological object of the test sample, insufficient information for decision making, or the like. In such cases, corresponding actions are needed to be performed to correct the error. The action indicated in a validation result for medical data is to suggest further validation of the medical data (referred to as a "second action"). The second action is a general suggestion, which means that the current medical data should not be released and manual review is required to decide how the medical data can be further validated.

In some examples, a machine learning model 212 can be trained to determine one or more specific actions that can be performed for further validation, including an action of re-running the medical test related to the medical data (referred to as a "third action"); an action of checking a historical patient medical record (referred to as a "fourth action"); an action of checking reaction of a reagent in the medical test (referred to as a "fifth action"), such as checking a reagent reacting curve; an action of checking a test sample collected for use in the medical test (referred to as a "sixth action"); an action of checking the medical data in combination with clinical diagnosis (referred to as a "seventh action"); and an action of checking patient drug use (referred to as an "eighth action"); and/or the like. It would be appreciated that the next-step actions listed above are merely some specific examples, and more, less, or different actions can also be specified as required in actual use cases.

By indicating one of those next-step actions in the validation result, it is easier and more efficient for the system or a laboratory technician to determine the next step to be performed in the medical diagnostic testing workflow. For example, if a validation result indicates a third action of re-running the medical test, the validation system 120 may automatically request the medical test system 110 in Fig. 1 to re-run the medical test so as to update the medical data. As the machine learning model 212 has learned from historical experience and knowledge to provide one of the actions, the requirements and pressures on the laboratory technician can be significantly reduced. The laboratory technician with limited experience and knowledge can be able to decide the next step of further validation without consuming much time in manually reviewing the medical data.

In some embodiments, the validation result from a machine learning model 212 may include an explicit indication of the action to be performed on the medical data and/or a confidence level of the machine learning model 212 in selecting the action for the current medical data. In determining the action to be output, the machine learning model 212 measures respective probabilities of the predetermined actions and select the one that has the highest probability. A probability of an action can indicate how confident the machine learning model 212 is with selecting the action as a next-step action. The confidence level may be considered as a risk rating of the action. The confidence level can also be used as a reference for the laboratory technician or the system to confirm whether the suggested action is suitable for the medical data 112.

The input (i.e., the medical data) and the output (i.e., the validation result) of the machine learning model 212 have been described. The training phase of machine learning implemented in the computing device 210 is to make the machine learning model(s) 212 to learn the association between the input and the output. The machine learning model(s) 212 are trained in a supervised manner based on training data 202 from a database 201. The training data 202 includes historical medical data and labeling information associated therewith. The historical medical data may include a number of medical test reports that are generated in different medical tests for one or more patients. The labeling information indicates respective actions performed on the medical test reports. As such, the labeling information may be used as ground-truth indications of the actions to be performed on the historical medical data. Generally, the labeled actions for the historical medical data are those that were considered to be the right actions for the medical data and/or those that are marked manually by the laboratory experts.

The machine learning model(s) 212 are trained to provide a validation result that indicates the same or similar action for input medical data as indicated in the associated labeling information. As such, the trained machine learning model(s) 212 can represent the association between the medical data and the validation results. The training of the machine learning model(s) 212 by the computing device 210 will be discussed in detail below.

The generated machine learning model(s) 212 may be provided by the computing device 210 to the computing device 230 via a communication network or may be stored by the computing device 210 to storage accessible by the computing device 210. The computing device 210 may utilize one or more of the generated machine learning models 212 to validate medical data. The computing device 210 is configured to obtain the medical data 112 to be validated and apply the medical data 112 to one or more machine learning models 212 to obtain the validation result 122 for the medical data 112.

The medical data 112 may sometimes be referred to as target medical data, which may be retrieved from storage or received from the medical test system 210. The validation result 122 may sometimes be referred to as a target validation result, which indicates information about a target action selected from the plurality of predetermined actions to be performed on the target medical data. The validation result 122 may include an indication of the target action and/or a confidence level of the machine learning model(s) 212 in selecting the target action. The computing device 230 may be present the validation result 122 to a user (such as a laboratory technician) or store the validation result 122 for further use. The application of the machine learning model(s) 212 by the computing device 230 will be discussed in detail below.

### Training of Machine Learning Model

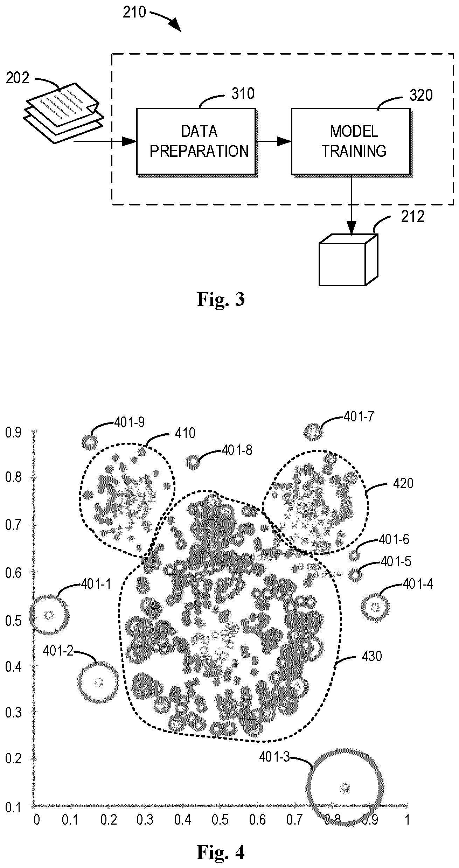

Fig. 3 illustrates a block diagram of architecture of implementing training of one or more machine learning models 212 in the computing device 210. As shown, the computing device 210 includes a data preparation module 310 to pre-process the training data 202 and a model training module 320 to implement the model training process based on the training data prepared by the data preparation module 310. In Fig. 3, one trained machine learning model 212 is depicted for purpose of illustration only. More than one machine learning model 212 can be trained in some cases.

In real-world scenarios, a large percentage of the medical test reports are validated by the laboratory experts as valid and can be released, which means that their associated labeling information may indicate the first action as mentioned above. Such medical data and the associated labeling information can be regarded as positive samples for the model training. Due to the large amount of available positive samples, it is desired to select those with higher reliability, which can ensure the accuracy of following machine learning.

Specifically, the data preparation module 310 is configured to obtain a set of available historical medical data (also referred to as a "first set of historical medical data") from the training data 202 that are marked as being associated with labeling information indicating the first action. The labeling information may be labeled to the first set of historical medical data manually by laboratory experts and/or automatically by some known available validation systems, such as the rule-based validation engine. The data preparation module 310 may further filter, from the first set of historical medical data, historical medical data that has higher reliability in the labeling information than other historical medical data in this set, and provide the selected historical medical data and associated labeling information for the model training module for training the machine learning model(s) 212.

The reliability of the associated labeling information may depend on various factors, for example, data sources from which the historical medical data and/or labeling information from are collected, conditions of the patients to which the historical medical data and/or labeling information are related, and/or the like. For example, the labeling information are determined to be have higher reliability if the historical medical data and labeling information are collected from the physical examination centers where a relatively larger number of patients to be examined are in health as compared with those from different clinics. In addition, the labeling information of the historical medical data that are collected from general clinics may have higher reliability than those from some intensive care departments. As a further example, ages, diagnosed diseases, professions, and/or other aspects of the patients may be set as certain criteria to filter out the labeling information of higher reliability. The historical medical data and labeling information related to young adults may be selected as compared with those related to elders.

In addition to the positive samples, negative samples, i.e., historical medical data with labeling information indicating other actions than the first action, may also be required for training the machine learning model(s) 212. In some conditions, the percentage of medical data labeled as requiring further validation (for example, any one of the actions other than the first action) is low due to the large amount of medical data that can be directly released. Although manual labeling is a possible choice to obtain sufficient training data of the negative samples, its main disadvantages are high cost and time consumption. In some embodiments, the data preparation module 310 may be configured to facilitate labeling of some historical medical data in the training data 202, especially to determine the labeling information to indicate the second general action of further validating the medical data.

The data preparation module 310 may utilize an unsupervised method to facilitate selection and labeling of the negative samples for training. Specifically, the data preparation module 310 is configured to select outlier historical medical data from a set of available historical medical data (referred to as a "second set of historical medical data"). Some of the second set of historical medical data may have been marked with associated labeling information, for example, the labeling information indicating the first action. In some examples, the second set of historical medical data may be obtained from part or all of the training data 202 stored in the database 201. The outlier historical medical data may be those that have outlier values of test results for some test items. Generally speaking, medical data with outlier values have higher probabilities of requiring further validation and thus may be likely associated with the second action.

The data preparation module 310 may be configured to cluster the second set of historical medical data to find the outlier historical medical data. For example, Fig. 4 shows an exemplary clustering result for a set of historical medical data. It is noted that in this example, for purpose of illustration only, each piece of historical data (for example, each historical medical test report) is shown to include two-dimensional data. Three clusters 410, 420, and 430 are formed after clustering the historical medical data. A number of pieces of outlier medical data 401-1 to 410-9 are away from the centroid of the three clusters and thus can be considered as outlier historical medical data. It would be appreciated that the example shown in Fig. 4 is provided merely for purpose of illustration without suggesting any limitation to the scope of the present disclosure.

Some or all of the outlier historical medical data may be marked with labeling information indicating the second action of requiring further validation. In some embodiments, the data preparation module 310 is configured to present the outlier historical medical data to a user for cross-labeling. The outlier historical medical data may be presented via a user interface of the computing device 230 or other terminal devices. Upon receiving a user input indicating one of the predetermined actions, the data preparation module 310 is configured to mark the outlier historical medical data to be associated with labeling information of the indicated action.

The user may explicitly specify a plurality of predetermined actions for the outlier historical medical data with his/her experience and knowledge. In this case, the user is allowed to mark the associated labeling information to indicate all the other actions than the first action. In some other examples, simple user confirmation is required to confirm whether the outlier historical medical data can be labeled with the second action or not. The data preparation module 310 may then determine the outlier historical medical data and associated labeling information as the negative samples for use in the model training. By filtering outlier historical medical data only for manual labeling, lower manual efforts and lower time consumption and costs are required in the data preparation phase.

In some embodiments, a machine learning model 212 may be trained to validate medical data including test results of a relatively large number of test items. However, historical medical data in some medical test reports may include past test results for some but not all of the test items in question, which leads to high sparsity since few medical test reports cover all the test items concerned by the machine learning model 212. To deal with the data sparsity, the data preparation module 310 may be configured to further process the training data to be used, for example, the training data obtained after the determination of positive and negative samples as described above.

The data preparation module 310 may be configured to process the historical medical data by filling test results for other test items that have no actual values. The filled test result for each test item may be determined from actual test results of that test item comprised in other historical medical data. For example, the filled test result for a test item may be calculated as median or mean of a reference range for each test item, where the reference range is determined from actual values of actual test results for the test item. The reference range may also be determined as a nominal reference range for the test item. As an alternative, the data preparation module 310 may transform high dimensional yet sparse historical medical data to lower dimensional but dense historical medical data by using dimensionality reduction techniques such as singular value decomposition (SVD) and principle component analysis (PCA).

Although the historical medical data and labeling information in the negative samples can be obtained as described above, the amount of available negative samples may still be less than the amount of positive samples (of which the labeling information indicates the first action), resulting in imbalanced training data. To control the imbalance, the data preparation module 310 may be configured to restrict the ratio of negative and positive samples in training of each machine learning model 212 to a reasonable level.

Specifically, the data preparation module 310 may be configured to select, from a set of whole available historical medical data (referred to as a "third set of historical medical data"), first historical medical data and second historical medical data based on a predetermined ratio of an amount of the first medical data to an amount of the second medical data. Here the "first historical medical data" refers to historical medical data associated with the labeling information that indicates the first action, and the "second historical medical data" refers to historical medical data associated with the labeling information that indicates a different action in the predetermined actions than the first action. The ratio of the selected first medical data and the selected second medical data may be equal to or lower than the predetermined ratio. The predetermined ratio can be set as any reasonable value depending on actual requirements in model training, for example, 10: 1, 5:1, 20:1, or the like. The data preparation module 310 may randomly sample from the whole available training data the first and second historical medical data.

The data preparation in the training phase has been discussed above. The training data 202 determined after the processing of the data preparation module 310 may be provided to the model training module 320. The model training module 320 may be configured to train one or more machine learning models 212. In an embodiment, the training data 202 from the data preparation module 310 may be split into different sets of training data to train different machine learning models 212. In a further embodiment, the same training data 202 may be used to train two or more different machine learning models 212. Each of the machine learning models 212 represents a different association between the medical data and the possible validation results. Different types of machine learning and/or different model configurations for a same type of machine learning can be utilized to build different machine learning models. For the same type of machine learning, different configurations are possible by choosing different hyperparameters when designing the models. The utilization of multiple machine learning models can be beneficial from taking advantages of different types of models and exploring different possible associations between the medical data and their validation results based on the different model configurations.

As mentioned above, a machine learning model 212 is expected to be trained to identify a target action from a plurality of predetermined actions to be indicated in the validation result 122. In some embodiments, a machine learning model 212 may be designed as a classification model for classifying/assigning the medical data into one of the classes corresponding to the plurality of predetermined actions. A machine learning model 212 that implements classification is referred as a classifier.

Examples of classification models are provided herein, e.g., decision trees, Bayesian models, random forest models, support vector machines, K-nearest neighbor (KNN) models, neural networks, and the like. Depending on the total number of possible classes (i.e., the number of actions to be performed on medical data), a machine learning model 212 may be based on binary classification or multi-class classification. In some embodiments, a machine learning model 212 may be built by integrating multiple learning algorithms (for example, neural networks or decision trees) to improve predictive performance of a single classifier.

To perform supervised machine learning, the validation results are required to be known for the historical medical data. A machine learning model 212 needs to learn from a vast amount of training data to predict or classify what new medical data should be categorized. However, labeling a large amount of data in order to train a model is time-consuming and is involved with lots of manual efforts. As a result, in some embodiments, the machine learning model(s) 212 may be designed especially for a small training dataset, which enables it to gather as much information as possible to distinguish from the multiple actions.

The model training module 320 may employ various training methods, either existing or to be developed in the future, in training the machine learning model(s) 212. The scope of the embodiments of the present disclosure is not limited in this regard. During the model training process, the model training module 320 may update parameters of the machine learning model(s) 212 iteratively until the model(s) 212 can represent the association between the medical data and the validation results, for example, can map input historical medical data correctly or almost correctly to the validation result indicating the ground-truth action as indicated in the labeling information.

In some embodiments, the model training module 320 or other module in the computing device 210 (not shown) may be configured to perform cross-validation on the machine learning model(s) 212 trained by the machine training module 320 to ensure that the model(s) 212 can get satisfied performance on the training data without over-fitting. Various methods for model cross-validation can be applied and the scope of the embodiments of the present disclosure is also not limited in this regard.

### Application of Machine Learning Model

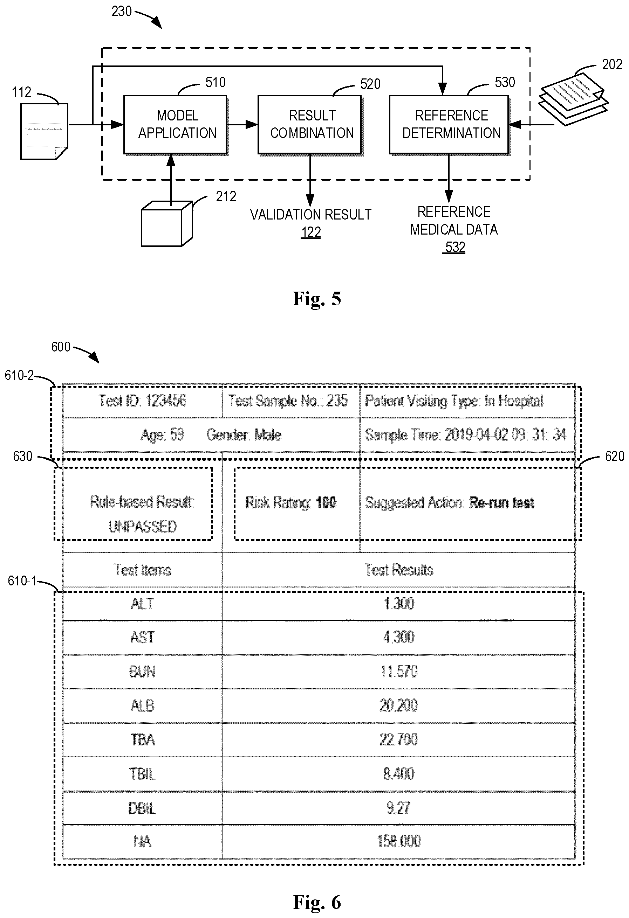

Fig. 5 illustrates a block diagram of architecture of implementing application of one or more trained machine learning models 212 in the computing device 230. In Fig. 5, one trained machine learning model 212 is depicted for purpose of illustration only. As shown, the computing device 230 includes a model application module 510 to obtain the machine learning model(s) 212 trained by the computing device 210 and then apply the target medical data 112 to the obtained machine learning model(s) 212. The target medical data 112 is provided as an input to each machine learning model 212. The machine learning model(s) 212 then process the target medical data 112 and associate it to an action of the predetermined actions. Each of the machine learning model(s) 212 then generates a validation result which includes an indication of the action and/or a confidence level of the machine learning model 212 in selecting that target action.

The computing device 230 also includes a result combination module 520 to provide the target validation result 122 for the target medical data 112. If one machine learning model 212 is utilized, the validation result output by this model may be directly provided as the target medical result 122. If two or more machine learning models 212 are utilized, the result combination module 520 determines the target validation result 122 based on the respective validation results from those models 212.

In some embodiments, the result combination module 520 may calculate a weighted average of the validation results, for example, by averaging values indicating the confidence levels included in the respective validation results. If the validation results indicate different actions to be performed on the target medical data 112, weighted averages for different actions may be determined. The result combination module 520 may determine the target validation result 122 to indicate the same action predicted by all the used machine learning models 212 and/or to indicate the weighted average as the confidence level. In the case of two or more possible actions being predicted, the result combination module 520 may compare the weighted averages with each other to select the higher weighted average and/or the corresponding action to be indicated in the target validation result 122. As an alternative, instead of calculating the weighted average, the result combination module 520 may directly select the validation result that is determined by the machine learning model 212 as having a higher confidence level than those indicated in other validation result. The result combination module 520 then determines the selected validation result as the target validation result 122.

The target validation result 122 and the target medical data 112 may be stored in association with each other. If the target validation result 122 indicates information about a target action other than the first action, which means that a certain type of further validation is required for the target medical data 112, then the target validation result 122 and the target medical data 112 may be presented together to the user to guide the next step of operation.

Fig. 6 shows an exemplary medical test report 600 which presents a medical data part 610-1 of the test results of corresponding test items, a medical data part 610-2 of the auxiliary information, and a validation result part 620 indicating the next-step action and the confidence level regarding this next-step action. As shown, it is suggested by the machine learning model(s) 212 to re-run the medical test, and the confidence level (i.e., the risk rating) for such suggestion is high (a score of 100). Generally, the confidence level calculated by a machine learning model is in a range between 0 to 1. To better show the risk, the confidence level is mapped to a range from 0 to 100 in the example of Fig. 6. It would be appreciated that the confidence level may be indicated in any other value range or by a non-numerical indicator.

In some embodiments, if the computing device 210 has trained a plurality of machine learning models 212 that are available for use, the computing device 230, such as the model application module 520 included therein, may be configured to select one or more machine learning models 212 for use based on respective performance measures of those models. The performance measures may be determined in the validation/test phase based on the training data, for example, by the computing device 210. Of course, the computing device 230 may calculate the performance measures for itself. In some embodiments, the performance measures of the machine learning models 212 may be calculated at the computing device 210 and then provided to the computing device 230.

The performance of each trained machine learning model 212 may be measured based on different characteristics associated with the model. In some embodiments, a receiver operating characteristic (ROC) curve and/or an area under the curve (AUC) may be calculated in the validation phase to indicate the performance of the model 212. In statistics, an ROC curve is a plot of the true positive rate (TPR) against the false positive rate (FPR) for different decision threshold. The true positive rate is also known as sensitivity or recall. The false positive rate is 1-specificity. An ROC curve can evaluate the discrimination ability of a classifier in terms of sensitivity and specificity and identify the optimal decision threshold. The ROC curve for a model with no discrimination capacity would be a 45-degree diagonal line. The area under the curve (AUC) is the probability that a classifier scores higher for randomly selected positive observation than a randomly selected negative observation. AUC can summarize the performance of a classifier with a single value.

Alternatively, or in addition, a confusion matrix of the machine learning model can be determined as a performance measure. The confusion matrix is a table that summarizes the prediction accuracy on a classification problem. Each row of the table represents the number of instances in each predicted class and each column represents the number of instances in each truth classes. A confusion matrix can also show the accuracy of predictions for each class.

In addition to provide the target validation result 122 for the target medical data 112 or as an alternative, the computing device 230 may further provide other medical data with similar properties as the target medical data 112 to the user, which can help and guide the user, especially junior technician or doctors, in reviewing and understanding the target medical data and/or the validation result. Referring back to Fig. 5, the computing device 230 includes a reference determination module 530 which is configured to provide such reference medical data.

Specifically, the reference determination module 530 is configured to a similarity between the target medical data 112 and candidate medical data. The candidate medical data may have been labeled or assigned with information about one of the predetermined actions that has been performed or is expected to be performed. Such candidate medical data may be selected from historical medical data that is used to generate the machine learning model 212, such as those included in the training data 202 as illustrated in Fig. 5. Alternatively, or in addition, the candidate medical data may also be selected from historical medical data that has been applied to the machine learning model 212 previously.

To narrow down the medical data that can be selected as the candidate medical data and to speed up the determination of the reference medical data, in some embodiments, the candidate medical data may be selected as having the same target action to be performed thereon and/or having the same types of test items as the target medical data. Such candidate medical data is possible to be considered as a more significant and meaningful reference for the target medical data 112.

In some embodiments, the reference determination module 530 may determine the similarity by calculating one or more test item similarities between one or more test results of test items indicated in the target medical data and one or more test results of test items indicated in the candidate medical data. The test item similarity may be measured by the Euclidean distance, which is based on a mathematical formula that computes a distance between two data points (i.e., two test results). The Euclidean distances between multiple pairs of test results may be combined (for example, weighted and summed) to represent the test item similarity. Any other algorithms can be employed in other examples to measure a distance between two data points.

If the similarity exceeds the predetermined similarity threshold, the reference determination module 530 may select the candidate medical data as reference medical data 532 for the target medical data 112. In some embodiments, the candidate medical data may be a medical test report in one medical test, and similarities between the target medical data 112 and other candidate medical data (other medical test reports) are calculated. In this case, the reference determination module 530 may rank the candidate medical data based on the similarities and select the candidate medical data that are ranked at high positions.

The similarity measurement may be performed in real time or may be pre-calculated. The pre-calculated similarity measurement may be triggered by certain criteria, for example, daily at night when no computing resource is used. For the pre-calculated similarity measurement, the reference determination module 530 may calculate the similarities among all the historical medical data and/or the previously-validated medical data, and then split the medical data into different clusters. In the case that new medical data is received and validated, the reference determination module 530 may only determine the similarities between the new medical data and the clusters (such as the medical data in the centroids of the clusters). The new medical data may be categorized into one of the clusters based on the similarities (for example, the one with the relatively high or highest similarity with the new medical data), and the previous medical data may be used as the reference medical data for the new medical data. In this way, the reference determination can be achieved in a timely manner.

### Evolution of Machine Learning Model

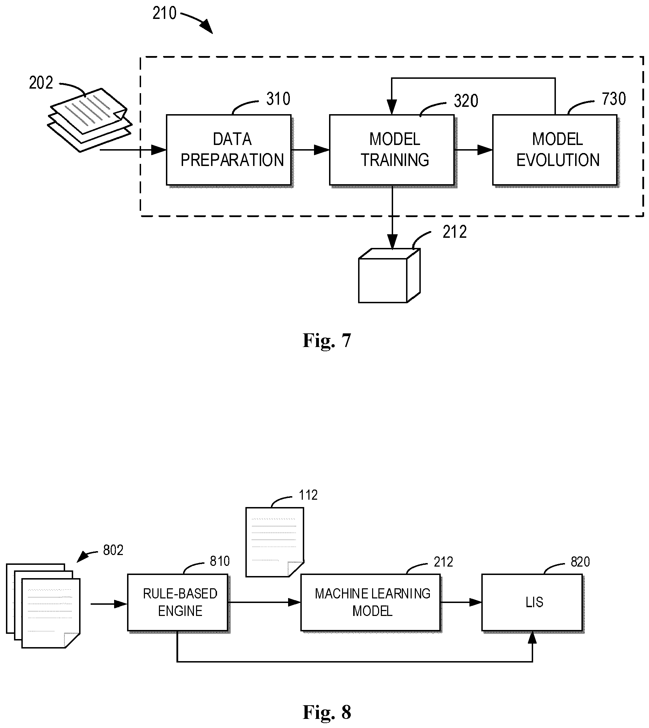

As the trained machine learning model(s) 212 are applied for a period of time and/or as requirements on the medical data validation change, it is expected to train new models to further improve the performances of the medical data validation. Fig. 7 illustrates a further example of the computing device 210 which further includes a model evolution module 730 to determine one or more further machine learning models 212 for validating medical data.

The evolution of the machine learning model at the model evolution module 730 may be initiated based on one or more predetermined trigger. A trigger for the evolution of the machine learning model may be based on the available training data for model training. At the initial stage, the training data may be limited and thus one or more machine learning models 212 may be trained with the limited training data for use. With the application of the model(s) 212, new medical data can be collected and their labeling information can be determined automatically by the previous models and/or in a manual manner based on users' feedback. If the amount of new training data has reached a certain threshold, the evolution of the machine learning model can be initiated.

Alternatively, or in addition to the training data-based trigger, in some embodiments, according to actual requirements, one or more test items are to be performed in a medical test and thus their results are to be added in the generated medical data. In this case, a new machine learning model 212 is to be trained to learn how to classify medical data with such new test items. In some cases, one or more new actions may be identified by the laboratory experts as possible actions to be performed on medical data that are validated as abnormal or suspicious, and/or one or more existing actions may be identified as being unnecessary in the automated validation. As such, one or more new machine learning models 212 may be trained to output validation results indicating information about a target action from the updated set of actions. In this way, the machine learning models can be evolved to adapt to technical updates and experience accumulation in medical diagnostic testing. Some other possible triggers for the machine learning model evolution may include an explicit indication from the user (such as the laboratory technician/experts), a time-based trigger, and the like.

In some embodiments, if the model evolution module 730 is determined to trigger the model evolution, it may request the model training module 320 to determine a further machine learning model 212 by updating a machine learning model 212 that has been trained. The updating of the machine learning model 212 may be performed by using new training data to refine the model parameters. The model evolution module 730 may be further configured to determine a further machine learning model 212 by generating a new machine learning model based on the previously-obtained training data and/or new training data. The new machine learning model may be designed as having a different model configuration than the machine learning model(s) 212 that have been trained. The different model configuration may be set as having different hyperparameters than the trained machine learning model(s) 212 and/or being related to a different type of machine learning.

In the case that one or more new actions are added and/or one or more existing actions are to be canceled, a new model with a new model configuration is required, which may be designed to be trained to represent an association between the medical data and further validation results indicating the updated actions to be performed on the medical data. For example, due to the limited training data at the initial stage, one or more machine learning models 212 may be initially designed to implement only binary classification between the first action and the second action of generally suggesting further validation to ensure model accuracy. With the increasing of available training data, one or more further machine learning models 212 can be trained for multi-class classification.

In some embodiments, if the number of available machine learning models 212 are increased with the evolution, the machine learning model(s) 212 that are provided for use may be determined based on their performance measures. That is, not all the updated/new machine learning models 212 are directly provided to replace the previous machine learning model(s) 212 that are currently in use, but only the models 212 with high performance measures are suitable for use in order to improve the validation performance.

In some embodiments, if it is determined that a new/updated machine learning model 212 has a higher performance measure than the machine learning model 212 that is currently used at the computing device 230, the new/updated machine learning model can be provided to replace the previous machine learning model 212. The performance measure of the machine learning model can be determined in a similar way as discussed above. The decision of whether or not to replace the models in use may be made by the computing device 210 or the computing device 230.

### Compatibility of Machine Learning Model

The machine learning models 212 can be applied independently to validate medical data. In some embodiments, the machine learning models proposed in the present disclosure can also be easily compatible with legacy components/functions for medical data validation. In some legacy validation procedures, a rule-based engine, as mentioned above, is applied for validating medical data. In some cases, a LIS may also be involved for archiving, recording, presentation, and/or further processing of medical data and validation results. The machine learning models proposed herein can also be adapted to the legacy medical data validating environments.

Fig. 8 illustrates a block diagram of a system where one or more machine learning models 212 are compatible with a rule-based engine 810 and a LIS 820. In Fig. 8, one machine learning model 212 is depicted for illustration only and more than one machine learning model 212 can be compatible with the rule-based engine 810 and the LIS 820. It would be appreciated that in some other cases, the rule-based engine 810 or the LIS 820 can be omitted. In the example of Fig. 8, the rule-based engine 810 and/or the LIS 820 may be implemented in separate devices from the computing device 230, or may be integrated within the computing device 230.

Conventionally, the rule-based engine 810 and the LIS 820 may be coupled together to implement the medical data validation. The automated validation based on one or more machine learning models 212 proposed herein can be implemented as an independent system to connect between the rule-based engine 810 and the LIS 820, for example, by reusing current communication interfaces of the rule-based engine 810 and the LIS 820. Through an adapter module, the rule-based engine 810, the machine learning model 212 and the LIS 820 can be connected to process medical data and provide validation results.

As specifically shown in Fig. 8, medical data 802 to be validated may be first input to the rule-based engine 810 for validation based on one or more predetermined rules. If the medical data 802 is validated by the rule-based engine 810 that it can be released to the entity requesting the medical test, this validation result and the medical data can be provided to the LIS 820 then. If some medical data 802 is determined by the rule-based engine 810 as to be further validated (i.e., the medical data 802 cannot be released), the medical data 802 is provided as the target medical data 112 to the machine learning model 212 for further processing.

Through this process, the rule-based engine 810 is used to filter medical data that are reliable to be released and thus less medical date is processed by the machine learning model 212, which may in turn reduce the overall resource consumption as the execution of the model generally requires more resources. The filtering of the rule-based engine will not result in sacrificing performance in view of the fact that the fixed rules are generally set manually to have high conservativeness and the validation result indicating the action of releasing the medical data is thus believed to have higher reliability. In some embodiments, the validation result of the rule-based engine 810 may also be presented in a medical test report together with the validation result from the machine learning model 212. For example, referring back to Fig. 6, the medical test report 600 is shown to further present a validation result part 630. The validation result part 630 indicates that the rule-based engine 810 determines the medical data as "unpassed," thereby requiring further validation.

In some embodiments, the machine learning model 212 may be disabled by the computing device 230 according to actual requirements and the medical data validation is performed by the rule-based engine 810 only in this case. In some embodiments, the rule-based engine 810 may be disabled and thus all the medical data 802 can be validated through the machine learning model 212. The enabling and disabling of the machine learning model 212 and/or the rule-based engine 810 may be determined based on feedbacks from the users (such as the laboratory technician/experts).

In some embodiments, the communication with the LIS 820 may follow some predefined specifications. Generally, the LIS 820 can support reception of respective test results of test items in the medical data, each test result being considered as one data item in a medical test report containing the medical data. The target validation result determined by the machine learning model may be provided in association with the target medical data to the LIS 820. To meet the specifications supported by the LIS 820, the communication with the LIS 820 may be easily expanded such that the target validation result can be treated as a test result of a dummy customized test item (i.e., a new data item) contained in the medical test report. As such, there is no need to modify the specifications for the communication with the LIS 820 to support the special validation result from the machine learning model. The LIS 820 may parse this dummy test item to obtain the target validation result from the machine learning model 212.

The communications between the machine learning model 212/the rule-based engine 810 and the LIS 820 may be scheduled based on any message scheduling method, such as a message bus scheduling which can implement asynchronous decoupling processing. It is convenient to extend the processing performance of the system through the message bus scheduling and expand the processing capability by adding multiple message receivers at the LIS 820. In some embodiments of implementing the rule-based engine 810, one or more machine learning models 212 and the LIS 820 in a distributed system, each machine learning model 212 may be packaged as a machine learning model service through micro-services. As such, the service registration, service discovery, and service version management can be performed through a model factory.

### Example Processes

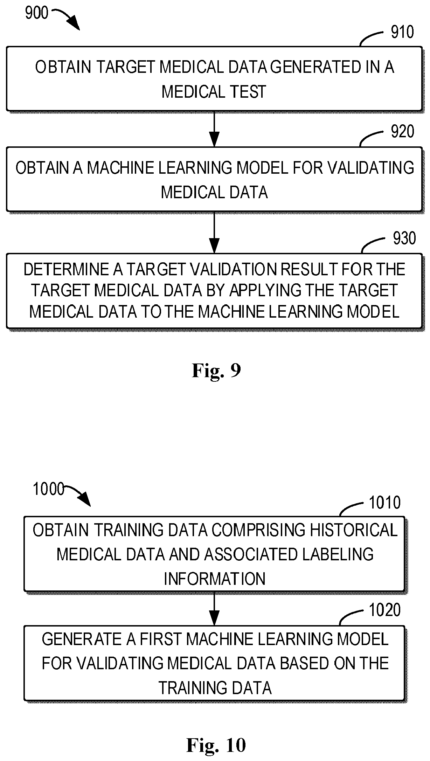

Fig. 9 illustrates a flowchart of an example process 900 for validating medical data according to some embodiments of the present disclosure. The method 900 can be implemented by the computing device 230 in Fig. 2. For the purpose of discussion, the method 900 will be described with reference to Fig. 2.

At block 910, the computing device 230 obtains target medical data generated in a medical test. At block 920, the computing device 230 obtains a machine learning model for validating medical data. The machine learning model represents an association between the medical data and validation results, and the validation results indicates information about predetermined actions to be performed on the medical data. At block 930, the computing device 230 determines a target validation result for the target medical data by applying the target medical data to the machine learning model. The target validation result indicates information about a target action selected from the predetermined actions to be performed on the target medical data.

In some embodiments, determining the target validation result comprises obtaining a further machine learning model for validating the medical data, the further machine learning model representing a different association between the medical data and the validation results than the association represented by the machine learning model; applying the target medical data to the machine learning model and the further machine learning model, respectively, to obtain respective validation results; and determining the target validation result based on the respective validation results.

In some embodiments, the process 900 further comprises determining a similarity between the target medical data and candidate medical data, the candidate medical data being selected from historical medical data that is used to generate the machine learning model, and/or historical medical data that has been applied to the machine learning model; in response to the similarity exceeding a predetermined similarity threshold, selecting the candidate medical data as reference medical data for the target medical data; and providing the reference medical data in association with the target medical data for presentation to a viewer of the target medical data.

In some embodiments, determining the similarity comprises selecting the candidate medical data based on at least one of the following: a determination that the target action is to be performed on the candidate medical data, and a determination that the candidate medical data have one or more test items that are the same as the target medical data.

In some embodiments, the predetermined actions comprise at least one of the following: a first action of releasing the medical data to an entity requesting a medical test related to the medical data, a second action of further validating the medical data, a third action of re-running the medical test related to the medical data, a fourth action of checking a historical patient medical record, a fifth action of checking reaction of a reagent in the medical test, a sixth action of checking a test sample collected for use in the medical test, a seventh action of checking the medical data in combination with clinical diagnosis, and an eighth action of checking patient drug use.

In some embodiments, the machine learning model comprises a classification model for classifying the medical data into classes corresponding to the predetermined actions.

In some embodiments, the information about the target action comprises at least one of the following: an indication of the target action, and a confidence level of selecting the target action for the target medical data by the machine learning model.

In some embodiments, the target medical data comprises medical data generated in an in-vitro diagnostic test.

In some embodiments, the machine learning model is selected from a plurality of available machine learning models based on respective performance measures of the plurality of available machine learning models.

In some embodiments, obtaining the target medical data comprises obtaining the target medical data that is determined by a rule-based engine as to be further validated, the rule-based engine being configured to validate the target medical data based on at least one predetermined rule.

In some embodiments, the process 900 further comprises providing the target validation result in association with the target medical data to a laboratory information system (LIS), the target medical data comprising at least one data item presented in a medical test report, and the target validation result presented in the medical test report as a further data item.

In some embodiments, the machine learning model is provided or trained by the process 1000 as will be described below with reference to Fig. 10.

Fig. 10 illustrates a flowchart of an example process 1000 for providing a machine learning model for validating medical data according to some embodiments of the present disclosure. The method 1000 can be implemented by the computing device 210 in Fig. 2. For the purpose of discussion, the method 1000 will be described with reference to Fig. 2.

At block 1010, the computing device 210 obtains training data comprising historical medical data and associated labeling information. The labeling information indicates predetermined actions performed on the historical medical data. At block 1020, the computing device 210 generates a first machine learning model for validating medical data based on the training data such that the first machine learning model represents an association between the medical data and validation results indicating information about the predetermined actions to be performed on the medical data.

In some embodiments, the predetermined actions comprise a first action of releasing the medical data to an entity requesting a medical test related to the medical data, and obtaining the training data comprises: obtaining a first set of available historical medical data that are marked as being associated with labeling information indicating the first action; selecting, from the first set of available historical medical data, historical medical data that has higher reliability in the labeling information than other historical medical data in the first set; and determining the selected historical medical data and the associated labeling information as the training data.

In some embodiments, obtaining the training data comprises: selecting outlier historical medical data from a second set of available historical medical data; presenting the outlier historical medical data to a user; in response to receiving, from the user, a user input indicating one of the predetermined actions, marking the outlier historical medical data to be associated with labeling information indicating the indicated action; and determining the outlier historical medical data and the associated labeling information as the training data.

In some embodiments, the predetermined actions comprise a first action of releasing the medical data to an entity requesting a medical test related to the medical data, and obtaining the training data comprises: selecting, from a third set of available historical medical data, first historical medical data and second historical medical data based on a predetermined ratio of an amount of the first medical data to an amount of the second medical data, the first historical medical data being associated with the labeling information that indicates the first action, and the second historical medical data being associated with the labeling information that indicates a different action in the predetermined actions than the first action.

In some embodiments, the process 1000 further comprises in response to a predetermined trigger for model evolution, determining a second machine learning model for validating medical data by: updating the first machine learning model, or generating a new machine learning model based on the training data, the new machine learning model having a different model configuration than the first machine learning model.

In some embodiments, determining the second machine learning model comprises: in response to determining that a further action is to be performed on medical data, adding, into the training data, further historical medical data and associated further labeling information indicating the further action; and generating the new machine learning model as the second machine learning model such that the second machine learning model represents an association between the medical data and further validation results indicating the predetermined actions and the further action to be performed on the medical data.

In some embodiments, the first machine learning model is provided in use for validating medical data, the method further comprising: determining a first performance measure of the first machine learning model and a second performance measure of the second machine learning model; and in response to the second performance measure exceeding the first performance measure, providing the second machine learning model to replace the first machine learning model in use.

In some embodiments, the historical medical data comprises a test result for at least one of a plurality of predetermined test items, and generating the first machine learning model comprises: processing the historical medical data by filling test results for other test items of the plurality of predetermined test items than the at least one test item, the filled test results being determined from test results of the other test items comprised in other historical medical data; and generating the first machine learning model based on the processed historical medical data.

In some embodiments, the predetermined actions comprise at least one of the following: a first action of releasing the medical data to an entity requesting a medical test, a second action of further validating the medical data, a third action of re-running the medical test related to the medical data, a fourth action of checking a historical patient medical record, a fifth action of checking reaction of a reagent in the medical test, a sixth action of checking a test sample collected for use in the medical test, a seventh action of checking the medical data in combination with clinical diagnosis, and an eighth action of checking patient drug use.

### Example Device

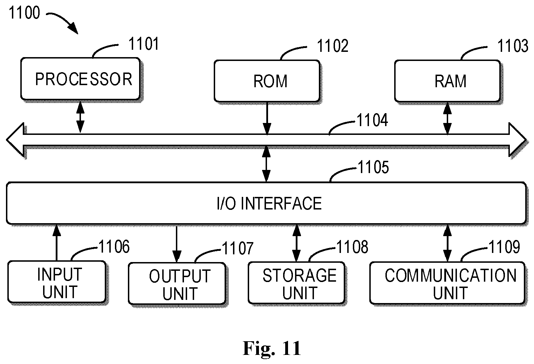

Fig. 11 illustrates a block diagram of an example computing system/device 1100 suitable for implementing example embodiments of the present disclosure. The system/device 1100 can be implemented as or implemented in the computing device 210 and/or the computing device 230 of Fig. 2. The system/device 1100 may be a general-purpose computer, a physical computing device, or a portable electronic device, or may be practiced in distributed cloud computing environments where tasks are performed by remote processing devices that are linked through a communication network. The system/device 1100 can be used to implement the process 900 of Fig. 9 and/or the process 1000 of Fig. 10.

As depicted, the system/device 1100 includes a processor 1101 which is capable of performing various processes according to a program stored in a read only memory (ROM) 1102 or a program loaded from a storage unit 1108 to a random access memory (RAM) 1103. In the RAM 1103, data required when the PROCESSOR 1101 performs the various processes or the like is also stored as required. The PROCESSOR 1101, the ROM 1102 and the RAM 1103 are connected to one another via a bus 1104. An input/output (I/O) interface 1105 is also connected to the bus 1104.

The processor 1101 may be of any type suitable to the local technical network and may include one or more of the following: general purpose computers, special purpose computers, microprocessors, digital signal processors (DSPs), graphic processing unit (GPU), co-processors, and processors based on multicore processor architecture, as non-limiting examples. The system/device 1100 may have multiple processors, such as an application-specific integrated circuit chip that is slaved in time to a clock which synchronizes the main processor.

A plurality of components in the system/device 1100 are connected to the I/O interface 1105, including an input unit 1106, such as keyboard, a mouse, or the like; an output unit 1107 including a display such as a cathode ray tube (CRT), a liquid crystal display (LCD), or the like, and a loudspeaker or the like; the storage unit 1108, such as disk and optical disk, and the like; and a communication unit 1109, such as a network card, a modem, a wireless transceiver, or the like. The communication unit 1109 allows the system/device 1100 to exchange information/data with other devices via a communication network, such as the Internet, various telecommunication networks, and/or the like.

The methods and processes described above, such as the process 900 and/or process 1000, can also be performed by the processor 1101. In some embodiments, the process 900 and/or process 1000 can be implemented as a computer software program or a computer program product tangibly included in the computer readable medium, e.g., storage unit 1108. In some embodiments, the computer program can be partially or fully loaded and/or embodied to the system/device 1100 via ROM 1102 and/or communication unit 1109. The computer program includes computer executable instructions that are executed by the associated processor 1101. When the computer program is loaded to RAM 1103 and executed by the PROCESSOR 1101, one or more acts of the process 900 and/or process 1000 described above can be implemented. Alternatively, PROCESSOR 1101 can be configured via any other suitable manners (e.g., by means of firmware) to execute the process 900 and/or process 1000 in other embodiments.

### Enumerated Example Embodiments

The embodiments of the present disclosure may be embodied in any of the forms described herein. For example, the following enumerated example embodiments describe some structures, features, and functionalities of some aspects of the present disclosure disclosed herein.

In a first aspect, example embodiments of the present disclosure provide a method for medical data validation. The method comprises obtaining target medical data generated in a medical test; obtaining a machine learning model for validating medical data, the machine learning model representing an association between the medical data and validation results, and the validation results indicating information about predetermined actions to be performed on the medical data; and determining a target validation result for the target medical data by applying the target medical data to the machine learning model, the target validation result indicating information about a target action selected from the predetermined actions to be performed on the target medical data.

In some embodiments, determining the target validation result comprises: obtaining a further machine learning model for validating the medical data, the further machine learning model representing a different association between the medical data and the validation results than the association represented by the machine learning model; applying the target medical data to the machine learning model and the further machine learning model, respectively, to obtain respective validation results; and determining the target validation result based on the respective validation results.

In some embodiments, the method further comprises determining a similarity between the target medical data and candidate medical data, the candidate medical data being selected from historical medical data that is used to generate the machine learning model, and/or historical medical data that has been applied to the machine learning model; in response to the similarity exceeding a predetermined similarity threshold, selecting the candidate medical data as reference medical data for the target medical data; and providing the reference medical data in association with the target medical data for presentation to a viewer of the target medical data.

In some embodiments, determining the similarity comprises: selecting the candidate medical data based on at least one of the following: a determination that the target action is to be performed on the candidate medical data, and a determination that the candidate medical data have one or more test items that are the same as the target medical data.

In some embodiments, the predetermined actions comprise at least one of the following: a first action of releasing the medical data to an entity requesting a medical test related to the medical data, a second action of further validating the medical data, a third action of re-running the medical test related to the medical data, a fourth action of checking a historical patient medical record, a fifth action of checking reaction of a reagent in the medical test, a sixth action of checking a test sample collected for use in the medical test, a seventh action of checking the medical data in combination with clinical diagnosis, and an eighth action of checking patient drug use.

In some embodiments, the machine learning model comprises a classification model for classifying the medical data into classes corresponding to the predetermined actions.

In some embodiments, the information about the target action comprises at least one of the following: an indication of the target action, and a confidence level of selecting the target action for the target medical data by the machine learning model.

In some embodiments, the target medical data comprises medical data generated in an in-vitro diagnostic test.

In some embodiments, the machine learning model is selected from a plurality of available machine learning models based on respective performance measures of the plurality of available machine learning models.

In some embodiments, obtaining the target medical data comprises: obtaining the target medical data that is determined by a rule-based engine as to be further validated, the rule-based engine being configured to validate the target medical data based on at least one predetermined rule.

In some embodiments, the method further comprises providing the target validation result in association with the target medical data to a laboratory information system (LIS), the target medical data comprising at least one data item presented in a medical test report, and the target validation result presented in the medical test report as a further data item.

In some embodiments, the machine learning model is provided by the method in the second aspect.

In a second aspect, example embodiments of the present disclosure provide a method of providing a machine learning model for validating medical data. The method comprises obtaining training data comprising historical medical data and associated labeling information, the labeling information indicating predetermined actions performed on the historical medical data; and generating a first machine learning model for validating medical data based on the training data such that the first machine learning model represents an association between the medical data and validation results indicating information about the predetermined actions to be performed on the medical data.

In some embodiments, the predetermined actions comprise a first action of releasing the medical data to an entity requesting a medical test related to the medical data, and obtaining the training data comprises: obtaining a first set of available historical medical data that are marked as being associated with labeling information indicating the first action; selecting, from the first set of available historical medical data, historical medical data that has higher reliability in the labeling information than other historical medical data in the first set; and determining the selected historical medical data and the associated labeling information as the training data.

In some embodiments, obtaining the training data comprises: selecting outlier historical medical data from a second set of available historical medical data; presenting the outlier historical medical data to a user; in response to receiving, from the user, a user input indicating one of the predetermined actions, marking the outlier historical medical data to be associated with labeling information indicating the indicated action; and determining the outlier historical medical data and the associated labeling information as the training data.

In some embodiments, the predetermined actions comprise a first action of releasing the medical data to an entity requesting a medical test related to the medical data, and obtaining the training data comprises: selecting, from a third set of available historical medical data, first historical medical data and second historical medical data based on a predetermined ratio of an amount of the first medical data to an amount of the second medical data, the first historical medical data being associated with the labeling information that indicates the first action, and the second historical medical data being associated with the labeling information that indicates a different action in the predetermined actions than the first action.

In some embodiments, the method further comprises in response to a predetermined trigger for model evolution, determining a second machine learning model for validating medical data by: updating the first machine learning model, or generating a new machine learning model based on the training data, the new machine learning model having a different model configuration than the first machine learning model.

In some embodiments, determining the second machine learning model comprises: in response to determining that a further action is to be performed on medical data, adding, into the training data, further historical medical data and associated further labeling information indicating the further action; and generating the new machine learning model as the second machine learning model such that the second machine learning model represents an association between the medical data and further validation results indicating the predetermined actions and the further action to be performed on the medical data.

In some embodiments, the first machine learning model is provided in use for validating medical data, the method further comprising: determining a first performance measure of the first machine learning model and a second performance measure of the second machine learning model; and in response to the second performance measure exceeding the first performance measure, providing the second machine learning model to replace the first machine learning model in use.

In some embodiments, the historical medical data comprises a test result for at least one of a plurality of predetermined test items, and generating the first machine learning model comprises: processing the historical medical data by filling test results for other test items of the plurality of predetermined test items than the at least one test item, the filled test results being determined from test results of the other test items comprised in other historical medical data; and generating the first machine learning model based on the processed historical medical data.

In some embodiments, the predetermined actions comprise at least one of the following: a first action of releasing the medical data to an entity requesting a medical test, a second action of further validating the medical data, a third action of re-running the medical test related to the medical data, a fourth action of checking a historical patient medical record, a fifth action of checking reaction of a reagent in the medical test, a sixth action of checking a test sample collected for use in the medical test, a seventh action of checking the medical data in combination with clinical diagnosis, and an eighth action of checking patient drug use.

In a third aspect, example embodiments of the present disclosure provide an electronic device. The electronic device comprises at least one processor; and at least one memory comprising computer readable instructions which, when executed by the at least one processor of the electronic device, cause the electronic device to perform the steps of the method in the first aspect described above.

In a fourth aspect, example embodiments of the present disclosure provide an electronic device. The electronic device comprises at least one processor; and at least one memory comprising computer readable instructions which, when executed by the at least one processor of the electronic device, cause the electronic device to perform the steps of the method in the second aspect described above.

In a fifth aspect, example embodiments of the present disclosure provide a computer program product comprising instructions which, when executed by a processor of an apparatus, cause the apparatus to perform the steps of any one of the methods in the first aspect described above.

In a sixth aspect, example embodiments of the present disclosure provide a computer program product comprising instructions which, when executed by a processor of an apparatus, cause the apparatus to perform the steps of any one of the methods in the second aspect described above.

In a seventh aspect, example embodiments of the present disclosure provide a computer readable medium comprising program instructions for causing an apparatus to perform at least the method in the first aspect described above. The computer readable medium may be a non-transitory computer readable medium in some embodiments.

In an eighth aspect, example embodiments of the present disclosure provide a computer readable medium comprising program instructions for causing an apparatus to perform at least the method in the second aspect described above. The computer readable medium may be a non-transitory computer readable medium in some embodiments.

Generally, various example embodiments of the present disclosure may be implemented in hardware or special purpose circuits, software, logic or any combination thereof. Some aspects may be implemented in hardware, while other aspects may be implemented in firmware or software which may be executed by a controller, microprocessor or other computing device. While various aspects of the example embodiments of the present disclosure are illustrated and described as block diagrams, flowcharts, or using some other pictorial representations, it will be appreciated that the blocks, apparatuses, systems, techniques, or methods described herein may be implemented in, as non-limiting examples, hardware, software, firmware, special purpose circuits or logic, general purpose hardware or controller or other computing devices, or some combination thereof.

The present disclosure also provides at least one computer program product tangibly stored on a non-transitory computer readable storage medium. The computer program product includes computer-executable instructions, such as those included in program modules, being executed in a device on a target real or virtual processor, to carry out the methods/processes as described above. Generally, program modules include routines, programs, libraries, objects, classes, components, data structures, or the like that perform particular tasks or implement particular abstract data types. The functionality of the program modules may be combined or split between program modules as desired in various embodiments. Computer-executable instructions for program modules may be executed within a local or distributed device. In a distributed device, program modules may be located in both local and remote storage media.

The computer readable medium may be a computer readable signal medium or a computer readable storage medium. A computer readable medium may include but is not limited to an electronic, magnetic, optical, electromagnetic, infrared, or semiconductor system, apparatus, or device, or any suitable combination of the foregoing. More specific examples of the computer readable storage medium would include an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing.

Computer program code for carrying out methods disclosed herein may be written in any combination of one or more programming languages. The program code may be provided to a processor or controller of a general purpose computer, special purpose computer, or other programmable data processing apparatus, such that the program codes, when executed by the processor or controller, cause the functions/operations specified in the flowcharts and/or block diagrams to be implemented. The program code may execute entirely on a computer, partly on the computer, as a stand-alone software package, partly on the computer and partly on a remote computer or entirely on the remote computer or server. The program code may be distributed on specially-programmed devices which may be generally referred to herein as "modules". Software component portions of the modules may be written in any computer language and may be a portion of a monolithic code base, or may be developed in more discrete code portions, such as is typical in object-oriented computer languages. In addition, the modules may be distributed across a plurality of computer platforms, servers, terminals, mobile devices and the like. A given module may even be implemented such that the described functions are performed by separate processors and/or computing hardware platforms.

While operations are depicted in a particular order, this should not be understood as requiring that such operations be performed in the particular order shown or in sequential order, or that all illustrated operations be performed, to achieve desirable results. In certain circumstances, multitasking and parallel processing may be advantageous. Likewise, while several specific implementation details are contained in the above discussions, these should not be construed as limitations on the scope of the present disclosure, but rather as descriptions of features that may be specific to particular embodiments. Certain features that are described in the context of separate embodiments may also be implemented in combination in a single embodiment. Conversely, various features that are described in the context of a single embodiment may also be implemented in multiple embodiments separately or in any suitable sub-combination.

Although the present disclosure has been described in languages specific to structural features and/or methodological acts, it is to be understood that the present disclosure defined in the appended claims is not necessarily limited to the specific features or acts described above. Rather, the specific features and acts described above are disclosed as example forms of implementing the claims.

## Claims

1. A method for medical data validation, comprising:
obtaining target medical data generated in a medical test;
obtaining a machine learning model for validating medical data, the machine learning model representing an association between the medical data and validation results, and the validation results indicating information about predetermined actions to be performed on the medical data; and
determining a target validation result for the target medical data by applying the target medical data to the machine learning model, the target validation result indicating information about a target action selected from the predetermined actions to be performed on the target medical data.

2. The method of claim 1, wherein determining the target validation result comprises:
obtaining a further machine learning model for validating the medical data, the further machine learning model representing a different association between the medical data and the validation results than the association represented by the machine learning model;
applying the target medical data to the machine learning model and the further machine learning model, respectively, to obtain respective validation results; and
determining the target validation result based on the respective validation results.

3. The method of any of claims 1 and 2, further comprising:
determining a similarity between the target medical data and candidate medical data, the candidate medical data being selected from historical medical data that is used to generate the machine learning model, and/or historical medical data that has been applied to the machine learning model;
in response to the similarity exceeding a predetermined similarity threshold, selecting the candidate medical data as reference medical data for the target medical data; and
providing the reference medical data in association with the target medical data for presentation to a viewer of the target medical data.

4. The method of claim 3, wherein determining the similarity comprises:
selecting the candidate medical data based on at least one of the following:
a determination that the target action is to be performed on the candidate medical data, and
a determination that the candidate medical data have one or more test items that are the same as the target medical data.

5. The method of any of claims 1 to 4, wherein the predetermined actions comprise at least one of the following:
a first action of releasing the medical data to an entity requesting a medical test related to the medical data,
a second action of further validating the medical data,
a third action of re-running the medical test related to the medical data,
a fourth action of checking a historical patient medical record,
a fifth action of checking reaction of a reagent in the medical test,
a sixth action of checking a test sample collected for use in the medical test,
a seventh action of checking the medical data in combination with clinical diagnosis, and
an eighth action of checking patient drug use.

6. The method of any of claims 1 to 5, wherein the machine learning model comprises a classification model for classifying the medical data into classes corresponding to the predetermined actions.

7. The method of any of claims 1 to 6, wherein the information about the target action comprises at least one of the following: an indication of the target action, and a confidence level of selecting the target action for the target medical data by the machine learning model.

8. The method of any of claims 1 to 7, wherein the target medical data comprises medical data generated in an in-vitro diagnostic test.

9. The method of any of claims 1 to 8, wherein the machine learning model is selected from a plurality of available machine learning models based on respective performance measures of the plurality of available machine learning models.

10. The method of any of claims 1 to 9, wherein obtaining the target medical data comprises:
obtaining the target medical data that is determined by a rule-based engine as to be further validated, the rule-based engine being configured to validate the target medical data based on at least one predetermined rule.

11. The method of any of claims 1 to 10, further comprising:
providing the target validation result in association with the target medical data to a laboratory information system (LIS), the target medical data comprising at least one data item presented in a medical test report, and the target validation result presented in the medical test report as a further data item.

12. A method of providing a machine learning model for validating medical data, comprising:
obtaining training data comprising historical medical data and associated labeling information, the labeling information indicating predetermined actions performed on the historical medical data; and
generating a first machine learning model for validating medical data based on the training data such that the first machine learning model represents an association between the medical data and validation results indicating information about the predetermined actions to be performed on the medical data.

13. The method of claim 12, wherein the predetermined actions comprise a first action of releasing the medical data to an entity requesting a medical test related to the medical data, and obtaining the training data comprises:
obtaining a first set of available historical medical data that are marked as being associated with labeling information indicating the first action;
selecting, from the first set of available historical medical data, historical medical data that has higher reliability in the labeling information than other historical medical data in the first set; and
determining the selected historical medical data and the associated labeling information as the training data.

14. The method of any of claims 12 and 13, wherein obtaining the training data comprises:
selecting outlier historical medical data from a second set of available historical medical data;
presenting the outlier historical medical data to a user;
in response to receiving, from the user, a user input indicating one of the predetermined actions, marking the outlier historical medical data to be associated with labeling information indicating the indicated action; and
determining the outlier historical medical data and the associated labeling information as the training data.

15. The method of any of claims 12 to 14, wherein the predetermined actions comprise a first action of releasing the medical data to an entity requesting a medical test related to the medical data, and obtaining the training data comprises:
selecting, from a third set of available historical medical data, first historical medical data and second historical medical data based on a predetermined ratio of an amount of the first medical data to an amount of the second medical data, the first historical medical data being associated with the labeling information that indicates the first action, and the second historical medical data being associated with the labeling information that indicates a different action in the predetermined actions than the first action.

16. The method of any of claims 12 to 15, further comprising:
in response to a predetermined trigger for model evolution, determining a second machine learning model for validating medical data by:
updating the first machine learning model, or
generating a new machine learning model based on the training data, the new machine learning model having a different model configuration than the first machine learning model.

17. The method of claim 16, wherein determining the second machine learning model comprises:
in response to determining that a further action is to be performed on medical data, adding, into the training data, further historical medical data and associated further labeling information indicating the further action; and
generating the new machine learning model as the second machine learning model such that the second machine learning model represents an association between the medical data and further validation results indicating the predetermined actions and the further action to be performed on the medical data.

18. The method of any of claims 16 and 17, wherein the first machine learning model is provided in use for validating medical data, the method further comprising:
determining a first performance measure of the first machine learning model and a second performance measure of the second machine learning model; and
in response to the second performance measure exceeding the first performance measure, providing the second machine learning model to replace the first machine learning model in use.

19. The method of any of claims 12 to 18, wherein the historical medical data comprises a test result for at least one of a plurality of predetermined test items, and generating the first machine learning model comprises:
processing the historical medical data by filling test results for other test items of the plurality of predetermined test items than the at least one test item, the filled test results being determined from test results of the other test items comprised in other historical medical data; and
generating the first machine learning model based on the processed historical medical data.

20. The method of any of claims 12 to 19, wherein the predetermined actions comprise at least one of the following:
a first action of releasing the medical data to an entity requesting a medical test,
a second action of further validating the medical data,
a third action of re-running the medical test related to the medical data,
a fourth action of checking a historical patient medical record,
a fifth action of checking reaction of a reagent in the medical test,
a sixth action of checking a test sample collected for use in the medical test,
a seventh action of checking the medical data in combination with clinical diagnosis, and
an eighth action of checking patient drug use.

21. The method of any of claims 1 to 11, wherein the machine learning model is provided by the method of any of claims 12 to 20.

22. An electronic device comprising:
at least one processor; and
at least one memory comprising computer readable instructions which, when executed by the at least one processor of the electronic device, cause the electronic device to perform the steps of any one of the methods according to claims 1 through 11 and claim 21.

23. An electronic device comprising:
at least one processor; and
at least one memory comprising computer readable instructions which, when executed by the at least one processor of the electronic device, cause the electronic device to perform the steps of any one of the methods according to claims 12 through 20.

24. A computer program product comprising instructions which, when executed by a processor of an apparatus, cause the apparatus to perform the steps of any one of the methods according to claims 1 through 11 and claim 21.

25. A computer program product comprising instructions which, when executed by a processor of an apparatus, cause the apparatus to perform the steps of any one of the methods according to claims 12 through 20.
